# EUROPEAN PATENT APPLICATION

(11) **EP 4 748 365 A1**
(43) Date of publication of application: **27.05.2026**
(21) Application number: 25215582.5
(22) Date of filing: 13.11.2025
(51) Int. Cl.: A61H 35/04

(54) **CLEANING DEVICE FOR NASAL CAVITIES WITH AN IMPROVED FILTRATION SYSTEM**

(30) Priority: 26.11.2024 IT 202400004917 U
(71) Applicant: FLAEM NUOVA S.p.A., 25015 BS Desenzano del Garda (IT)
(72) Inventor: ABATE, Riccardo, 25015 Desenzano del Garda (BS) (IT); ONGARETTI, Luca, 25015 Desenzano del Garda (BS) (IT); VENTURI, Alessandro, 25015 Desenzano del Garda (BS) (IT)
(74) Representative: Botti & Ferrari S.p.A.

(57) **Abstract**

The present invention relates to a cleaning device (1, 1') for nasal cavities comprising a hollow main body (2) comprising an air inlet (11) and defining a housing space (4), at least one respiratory care device (5, 35) for the upper or lower respiratory tracts, removably coupled to the main body (2) and an activation element (16).

The device (1, 1') further comprises a compressor unit (13) adapted to be inserted in said housing space (4) and comprising a hollow motor support (14) adapted to contain a compressor (15) and defined between a bottom (3) and an upper end (16) close to the housing of the respiratory care device (5, 35).

The main body (2), the compressor unit (13), and the at least one respiratory care device (5, 35), when assembled, define an inner suction chamber (17) comprising a flow inlet (18), a compressor flow inlet (20), and a flow outlet (19).

The motor support (14) comprises a channel (21), preferably longitudinal, along an outer wall (22) of the motor support (14) defined between the air inlet (11) and the flow inlet (18) of the suction chamber (17).

## Description

### Field of application

In its more general aspect, the present invention relates to a cleaning device for nasal cavities with an improved filtration system.

The present invention is generally, but not limitedly, applied in the field of respiratory tract care and more precisely of the upper and lower respiratory tracts.

### Prior art

In the present field, it is well known that, when suffering from diseases affecting the upper respiratory tract, it is advisable to adopt therapies aimed at eliminating mucus and bacteria.

This therapy is particularly important for children, since it improves breathing and reduces the risk of more serious infections or related conditions.

One of the most commonly used methods involves the use of a nasal douche to clear the nose, since it is able to thoroughly wash the nasal cavities removing mucus and bacteria by using a completely painless and minimally invasive method.

These nasal douches are usually carried out through saline solution, special thermal water, or drugs, thus employing non-aggressive substances which, when applied in this way, are highly effective.

Unlike the common nasal washes, these nasal douches are actual medical devices that, by nebulising a jet of liquid substances directly into the nasal cavities, dissolve mucus and maintain proper humidity inside the nose.

Keeping the nasal cavities clean prevents pathogens from attacking the inner walls, which are more vulnerable when dry.

In fact, inflammation of the respiratory tract mucosa leads to the proliferation of bacteria and viruses, which are normally repelled by the mucosa natural defences.

In this way, an inflammatory process can turn into an infectious condition.

This causes ailments ranging from colds, blocked noses and coughs to sinusitis, rhinitis, nasal polyposis, and adenoiditis.

Therefore, nasal douche is a tool that can be used both to treat upper respiratory tract disorders and to prevent them through constant personal hygiene.

Nasal douche can also be used in cases of otitis, since it has the effect of eliminating bacteria present in the nasopharyngeal tract and of protecting this area from further pathogenic attack.

This inflammatory process could also spread from the upper respiratory tract to the lower respiratory tract.

Therefore, therapies are aimed at eliminating mucus and bacteria through the use of a nasal douche, which allows cleaning the nose deep down, decongesting the upper respiratory tract to combat colds.

Similarly, nasal douche is used to keep the nose clean in order to increase resistance to all diseases affecting the respiratory tract.

The benefits of nasal douche are therefore numerous and significant, playing a decisive role in the healing and prevention of diseases of the upper respiratory tract.

Conventional nasal douches consist of a specially shaped ampoule and a specific nebulisation system that allows the washing liquid to flow into the nasal cavities, usually with the aid of a compressor.

Electrical devices comprising a pressurised reservoir containing the washing liquid associated with a nebulisation system are also known.

Furthermore, these nasal douches comprise a main body that can be held by a user.

An electrically powered compressor for pressurising the reservoir is connected to said main body.

Alternatively, for the treatment of the lower respiratory tract, a classic nebulisation ampoule is often used, with an upper component and a lower component connected to each other, an opening for inletting the nebulised medical mixture, an inlet for inletting air into the ampoule, and an atomiser for inletting pressurised gas into the lower component.

Although the current technical solutions are highly standardised, there are still some drawbacks to their use.

In particular, the healthiness of the ambient air fed into the respiratory care device, whether it is a nasal douche or a nebulisation ampoule, to allow the operation thereof, is not always guaranteed.

For this reason, there is a perceived need to introduce solutions that further improve the functionality of respiratory care devices compared to those currently available.

Therefore, an object of the present invention is to solve the prior art drawbacks and suggest an optimal and effective solution for domestic use.

An object of the invention is also to provide a solution suitable for multiple respiratory care devices.

Another object is to provide a solution that allows achieving the best results in terms of beneficial effects for a user.

A further object is to provide a compact and robust solution.

A further object of the invention is to provide a practical solution that does not imply complicated use.

Still an object of the invention is to provide a solution that is lightweight and resistant to cleaning and disinfection operations.

Still a further object is to provide feedback to the user on the operating status of the device.

Finally, an object of the present invention is to provide a solution that is suitable for a practical mass production.

### Summary

The idea underlying the present invention is to provide a solution that involves a specific flow path for the air entering the nasal douche or nebuliser, so as to ensure the operation thereof in optimal health conditions.

Based on this solution idea, the above objects are solved by a device comprising a main body comprising an air inlet and defining a housing space, a respiratory care device removably coupled to the main body, and an activation element.

The device further comprises a compressor unit adapted to be inserted into the housing space and comprising a hollow motor support adapted to contain a compressor and defined between a bottom of the main body and an upper end close to the care device for the upper or lower respiratory tracts.

The main body, the compressor unit, and the respiratory care device, when assembled, define an inner suction chamber comprising a flow inlet, a compressor flow inlet, and a flow outlet.

The motor support comprises a channel, preferably longitudinal, along an outer wall of the motor support defined between the air inlet and the flow inlet of the suction chamber.

Advantageously, in an assembled condition a flow path for the inlet air that starts from the air inlet is generated, it runs along the channel, arrives into the suction chamber and then into the nasal douche device or nebuliser, thus ensuring that the air passes through a defined and hermetic path, which allows preventing pollution problems typically caused by internal components, such as DC motor brushes or wear and tear on electrical components.

Preferably, the device according to the present invention further comprises an air filter element at the air inlet.

Advantageously, there is a further guarantee of healthiness of the inlet air, a condition that is kept in the airtight defined subsequent path.

More preferably, the device according to the present invention further comprises an air filter cap adapted to be inserted at the air filter element.

Advantageously, in unused conditions, the air filter is protected and therefore remains unaffected when not required.

Preferably, the device according to the present invention further comprises a control circuit associated with the compressor unit.

Advantageously, the control circuit makes the device according to the invention a "smart" device, to be managed based on specific needs.

Preferably, the motor support is made of natural polypropylene PP, adapted to guide light signals of the control circuit to an outer ring of the motor support.

Advantageously, said material is suitable for medical use, is particularly resistant, lightweight, and suitable for mass production, and also allows for the desired light diffusion of signals.

More preferably, the device according to the present invention further comprises a communication and/or power socket and, preferably, a socket cap.

Advantageously, the present solution allows a direct and intuitive interfacing with the present device by a user.

It is possible to provide for different types of connectors.

Still preferably, the device according to the present invention further comprises a lower motor support gasket at a bottom of the motor support and an upper motor support gasket at the suction chamber.

Advantageously, said solution avoids the presence of unwanted air leaks inside the device.

Preferably, the activation element comprises a first activation button on the main body and a second activation button to select a nebulisation speed.

Advantageously, the present solution enables a simple and intuitive activation of the device.

Still preferably, the respiratory care device is couplable to the main body by conical interference.

Advantageously, said solution ensures a stable coupling but also a possible separation without excessive effort by a user.

According to a first embodiment, the respiratory care device is a nasal douche device, in particular for the upper respiratory tract. The nasal douche device comprises a reservoir, a recovery chamber, a nebulisation system, and a nasal adapter at an outlet of the nebulisation system.

According to a second embodiment, the respiratory care device is an aerosol therapy ampoule, in particular for the lower respiratory tract.

According to another aspect of the present invention a compressor unit is provided for a cleaning device for nasal cavities, comprising a hollow motor support, a compressor inserted in the motor support, and a channel, preferably longitudinal, along an outer wall of the motor support.

Advantageously, said solution on the one hand allows making a pre-assembled unit tested in isolation before use and on the other hand a correct healthiness of air entering from outside.

The features and advantages of the cleaning device for nasal cavities according to the present invention will become clearer from the following description of an exemplifying embodiment thereof given by way of indicative and non-limiting example with reference to the appended drawings.

### Brief description of the drawings

In these drawings:

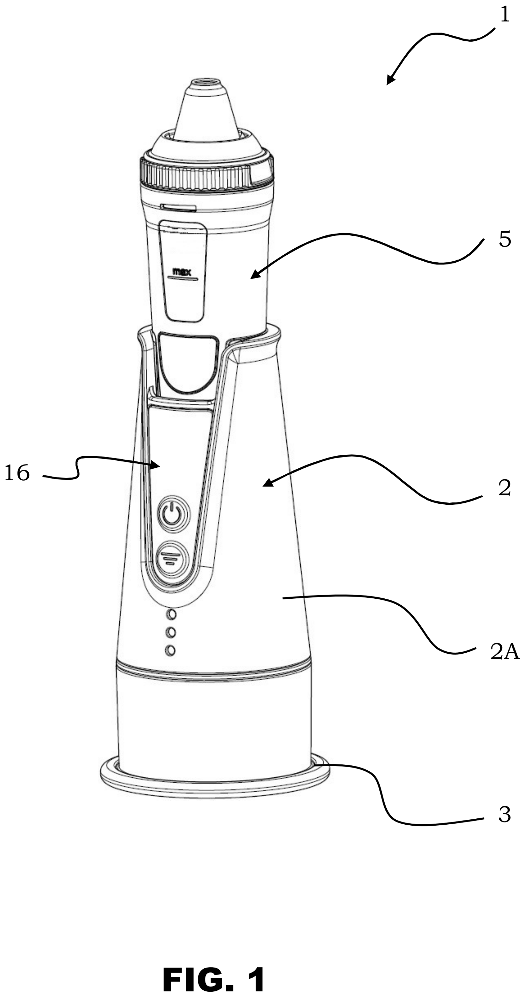

- Figure 1 shows a schematic perspective view of a cleaning device for nasal cavities according to the present invention coupled to a nasal douche device;

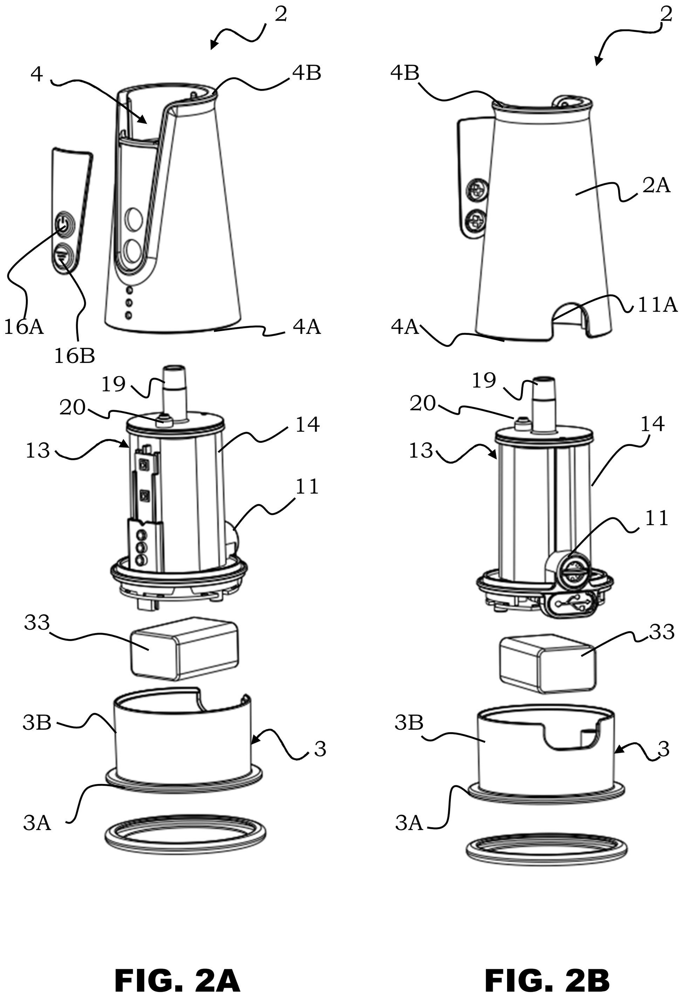

- Figures 2A, 2B show exploded schematic views of a portion of the device of Figure 1;

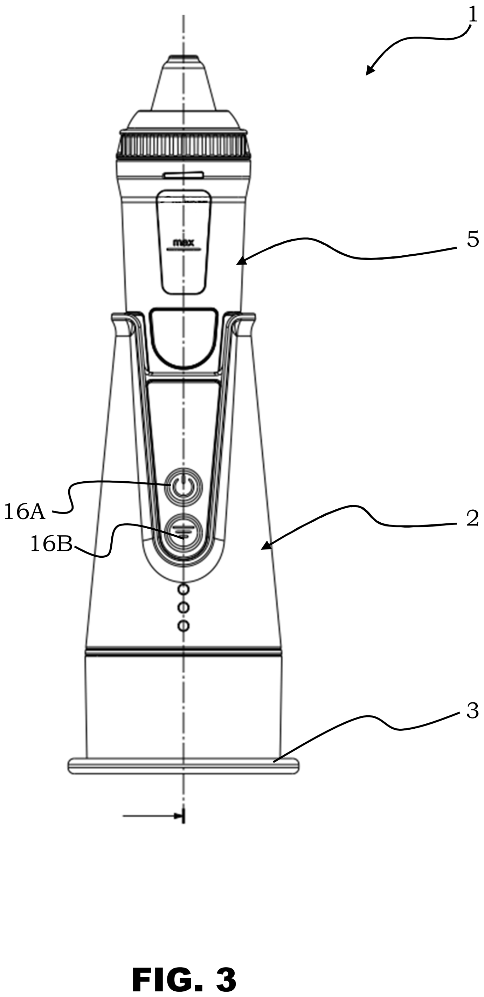

- Figure 3 shows a front schematic view of the device of Figure 1;

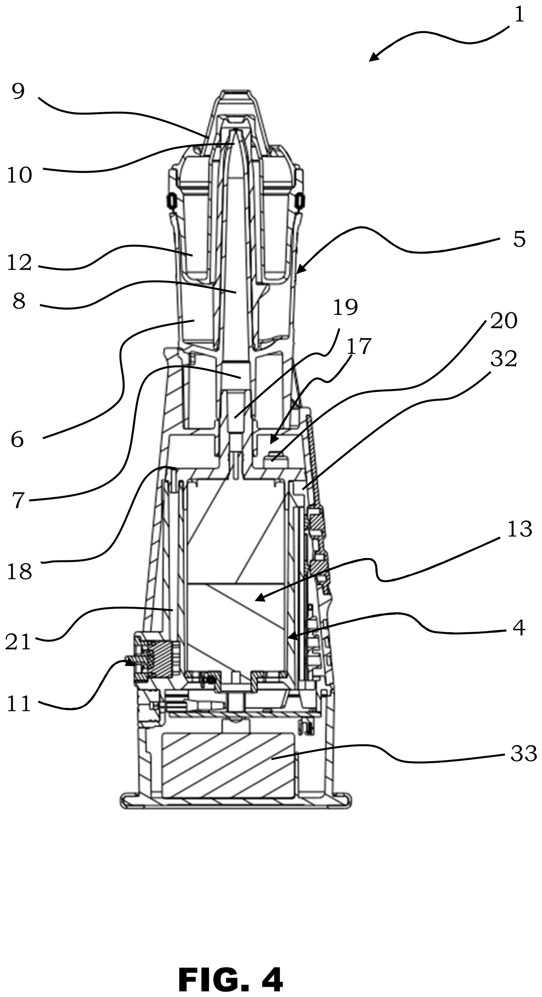

- Figure 4 shows a cross-sectional schematic view of Figure 3;

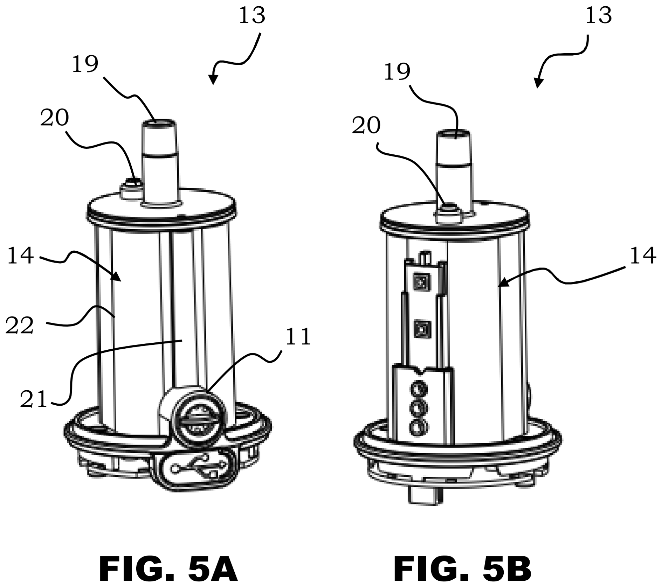

- Figures 5A, 5B show schematic views of a compressor unit of the device of Figure 1;

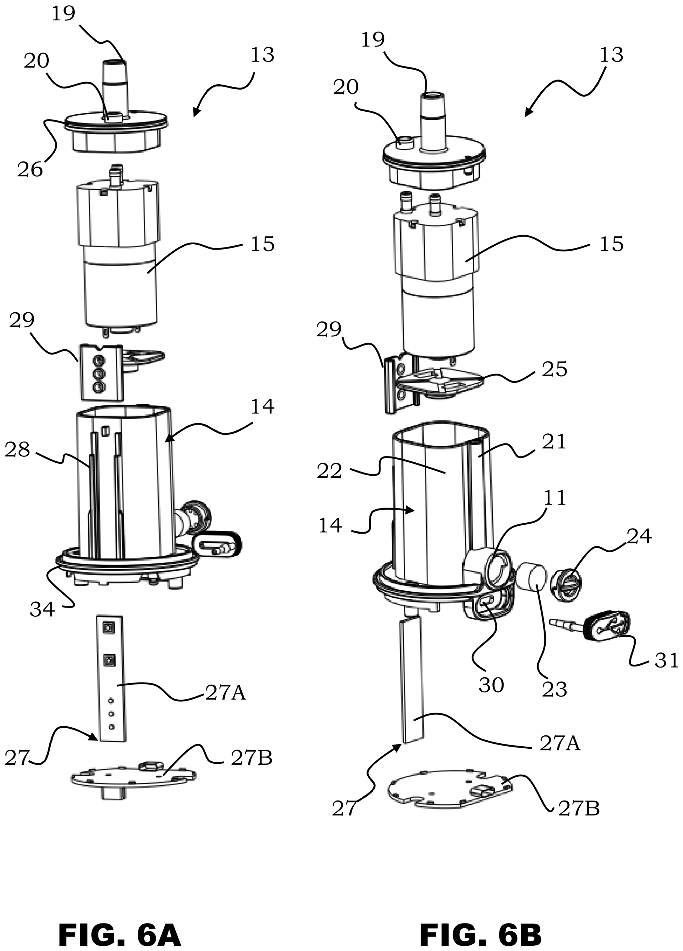

- Figures 6A, 6B show exploded schematic views of the compressor unit of Figures 5A, 5B.
- Figures 7-9 show schematic perspective view of a cleaning device for nasal cavities according to the present invention coupled to an aerosol therapy ampoule.

### Detailed description

With reference to these figures, reference numbers 1. 1' globally and schematically indicates a cleaning device for nasal cavities according to the present invention, hereinafter only referred to as device 1, 1' for the sake of brevity.

The device 1, 1' comprises a main body 2.

In the present embodiment the main body 2 is hallow and substantially cylindrical-shaped, defining a housing space 4 therein between lower end 4A and an upper opening 4B.

There is nothing to prevent the provision of a main body 2 with a different shape.

In the present embodiment, a bottom 3 is provided, which comprises a support base 3A and a peripheral protrusion 3B, the latter being adapted to shape-couple in said lower end 4A, so as to close the housing space 4 below.

The bottom 3 also provides a stable support for the device 1.

A respiratory care device is axially inserted and housed inside the upper opening 4B.

In a first embodiment, a device 1 comprising a nasal douche device 5, in particular for the upper respiratory tract, is provided.

More specifically, the nasal douche device 5 fits by central conical interference with the main body 2 inside the upper opening 4B, so as to ensure the tightness in operation without pressure loss and without loose or imprecise connections.

Nothing prevents from providing a non-axial coupling, for instance transversal, between main body 2 and nasal douche device 5, these variants being comprised in the scope of protection defined in the appended claims.

The nasal douche device 5 comprises a reservoir 6, into which the medical solution to be used for the patient is introduced, and functionally coupled with a conical fitting 7 that nebulizes the solution into microparticles with a diameter of a few microns.

The solution passes through a nebulisation system 8, comprising a duct preferably passing centrally axially, which carries the nebulized solution from the reservoir 6 up to a nasal adapter 9 placed on top of the nasal douche device 5 at the outlet 10 of the nebulisation system 8.

Furthermore, a recovery chamber 12 for the fluid downstream of the use of the nasal douche device 5 is provided.

According to the non-limiting embodiment represented, the nasal douche device 5 is provided with the reservoir 6 and the recovery chamber 12 in an overlapping configuration, so as to optimise the overall bulk of the device.

However, nothing prevents from providing different types of nasal douche devices with different internal features, these variants being comprised in the scope defined in the appended claims.

The nasal adapter 9 is generally, but not limitedly, removably coupled to the remaining portion of the nasal douche device 5, so as to ensure the decoupling and sterilisation thereof after each use.

The device 1 can be activated by means of an activation element 16.

In the embodiment represented, the activation element 16 is provided with activation buttons 16A and 16B on the side surface of the main body 2: the button 16A turns on and off, the button 16B allows selecting the nebulisation speed that varies the power absorbed by the compressor.

The user presses the first activation button 16A to activate the operation of the device 1 and the second activation button 16B to select the nebulisation speed and consequently the administration of nebulised medical solution generated between reservoir 6 and conical fitting 7, through the nebulisation system 8. The various speeds allow for more delicate or more decisive nebulisation depending on the user, for instance an adult or a child.

At the side surface 2A of the main body 2 an air inlet 11 is provided, which is made of a duct that is substantially transversal to said side surface 2A and passing through an opening 11A in said side surface 2A.

The device 1 also comprises an assembled compressor unit 13 adapted to be inserted into the housing space 4 and functionally coupled to the nasal douche device 5 to make the device 1 functionally autonomous and compact.

The compressor unit 13 comprises a hollow motor support 14 adapted to contain a compressor 15 and defined between the bottom 3 and an upper end 16 close to the nasal douche device 5.

In the preferred embodiment depicted, the compressor 15 is made of a diaphragm pump, therefore with a pneumatic valve that directs the compressed air back and forth between the two sides of the pump.

Nothing prevents from adopting a different technology, based on specific requirements or based on any future developments.

Nothing prevents from using different materials, these variants being comprised in the scope defined in the appended claims.

When assembled, said main body 2, the compressor unit 13 and the nasal douche device 5 define a suction chamber 17, inside the main body 2 close to the upper end 4B where the coupling with the nasal douche device 5 is provided, and comprising a flow inlet 18 at the suction chamber 17, a compressor flow inlet 20 into the pump 15, a flow outlet 19 from the pump 15 to the nasal douche 5, or, as it will be specified hereinafter, a different respiratory care device 35.

The motor support 14 comprises a channel 21 along an outer wall 22 of the motor support 14, preferably, but not limitedly, made integral and preferably longitudinal in the direction between lower end 4A and upper opening 4B of the main body 2.

Said channel 21 is defined between the air inlet 11 and the flow inlet 18 of the suction chamber 17.

In his way, the air entering the device 1 runs along a defined and hermetic path, that allows not having pollution problems generally caused by internal components, such as DC motor brushes or wear and tear on electrical components.

In other words, the air is sucked through the air inlet 11 and conveyed through the channel 21 preferably made integral with the motor support 14 up to the inner suction chamber 17 created by the assembly between the main body 2 of the device 1, the compressor unit 13, and the nasal douche device 5.

The air is then sucked from the compressor flow inlet 20 for the air flow entering the compressor 15 and exiting from the flow outlet 19 toward the nasal douche device 5.

The compressor unit according to the present invention for the device 1, comprising the hollow motor support 14, the compressor 15 inserted therein, and the channel 21, preferably longitudinal, along the outer wall 22 of the motor support 14 on the one hand allows making a pre-assembled unit tested in isolation before use and, on the other hand, a correct healthiness of the air entering from outside.

Still furthermore, to even more improve the quality of the air entering the device 1, in the embodiment represented an air filter element 23 is provided inside the air inlet 11 so that, in operation, the air that travels along the airtight flow path defined by the channel 21 has already been made perfectly healthy at the air inlet 11.

Moreover, to prevent the air filter element 23 from being affected in any way by pollutants when not in use, which would reduce its useful life cycle, in the present embodiment an air filter cap 24 is provided, which is to be placed at the air filter element 23 in these cases.

Still furthermore, the device 1 comprises a lower motor support gasket 25 at a bottom of the motor support 14 and an upper motor support gasket 26 at the suction chamber 17.

In this way, unwanted air leaks are avoided inside the device 1.

In particular, the upper motor support gasket 26 delimits the suction chamber 17 and also acts as adaptor for the respiratory care device placed at the exit.

It is preferably made of silicone, and radially interferes with the main body 2 of the device 1, thus creating an airtight chamber.

In other words, it is sealed by radial interference points 32 that guarantee a perfect seal.

In the present embodiment, a control circuit 27 associated with the compressor unit 13 is further provided, which makes the device 1 according to the invention a "smart" device, to be manged based on specific needs.

As visible in the appended figures, the control circuit 27 is provided with a plate-like element 27A associated with the motor support 14 at two track longitudinal protrusions 28 made on the outer wall 22 of the latter, made so as to allow a shape-coupling of said plate-like element 27A sliding between the two longitudinal protrusions 28.

The control circuit 27 is also provided with a base 27B at the bottom of the motor support 14 whereon the plate-like element 27A is fitted, on which lighting LEDs of the RGB type are installed.

Moreover, in the present embodiment, the motor support 14 is made of natural polypropylene PP, to allow good resistance to cleaning and disinfection operations and to guide the light signals of the control circuit to an outer ring 34 of the motor support at the bottom 3.

Moreover, a light guide 29 that is shape-coupled in an overlapping manner on the two longitudinal protrusions 28 is provided, which is made of a plate with coupling ends on said protrusions, with reflection points located on both faces of the plate-like element 27A and with other reflection points located in the central part of the plate-like element 27A, so as to diffusely reflect light over the surface thereof.

The outer wall 25 of the motor support has a shape that is also adapted to transmit the light emitted by the lighting LEDs of the RGB type toward the outer surface 25 of the motor support, to visually communicate the operating and charging status of the device to the user.

In the present embodiment, a battery 33 is further provided, which is housed in the housing space 4 at the bottom 3 made, as mentioned, as support base 3A and peripheral protrusion 3B.

A battery 33 of the rechargeable type or a battery 33 of the disposable type may be provided.

Nothing prevents the provision of a different cable power supply in alternative embodiments.

Moreover, in the depicted preferred embodiment a communication and/or power socket 30 is provided and, preferably, a socket cap 31 to protect the latter when not used.

Said communication and/or power socket 30 allows a direct and intuitive interfacing with the compressor unit 13 by a user and/or a battery 33 charging.

Different types of connectors may be provided. In the present embodiment a communication standard by means of USB is adopted, but nothing prevents from providing different connectors based on specific needs.

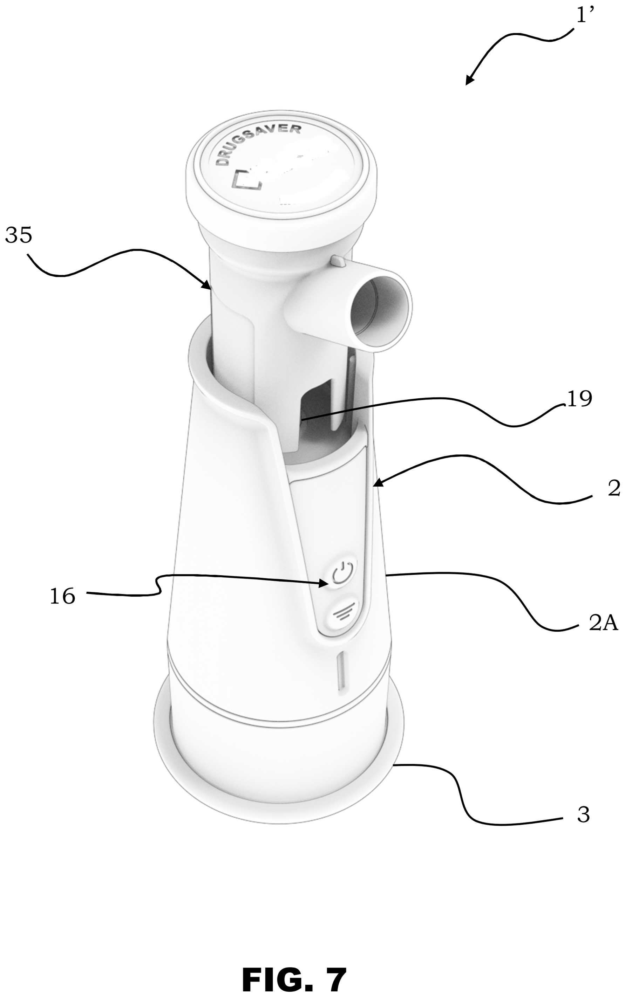

In Figures 7-9, a second embodiment of a device 1' according to the present embodiment is shown, which provides for the use of an aerosol therapy nebulisation ampoule 35 instead of the nasal douche device 5.

More particularly, in Figure 7 a respiratory care device 35 is shown, which is made of a nebulisation ampoule, in particular for the lower respiratory tract, directly coupled to the compressor unit 13 inserted in the main body 2, at the flow outlet 19 from the pump 15.

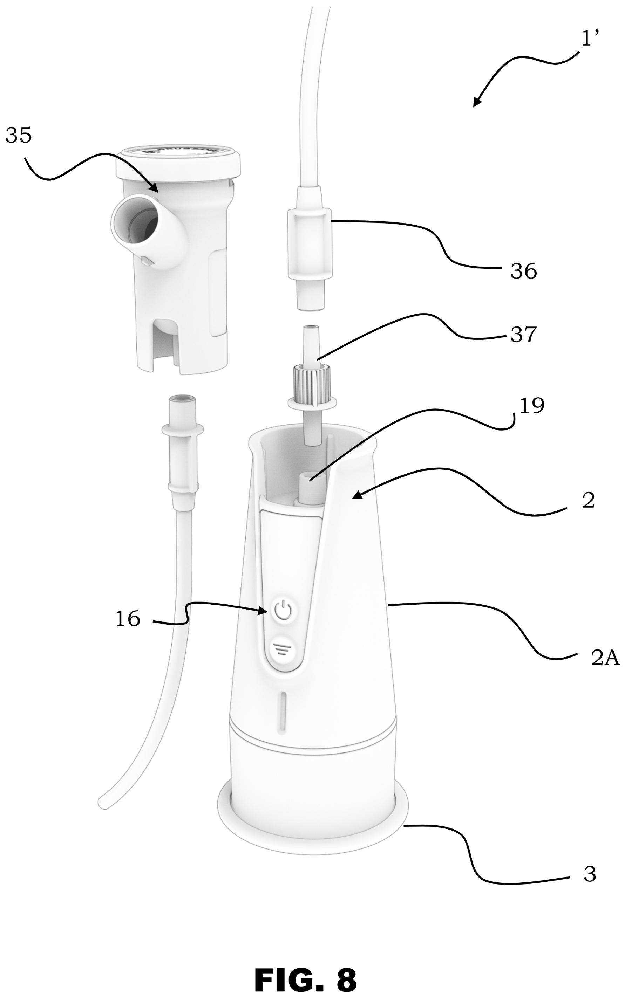

Alternatively, Figure 8 shows a nebulisation ampoule 35 coupled to the compressor unit 13 inserted in the main body 2 through a tube 36, preferably made of PVC, and a proper adaptor fitting 37 at the flow outlet 19 from the pump 15.

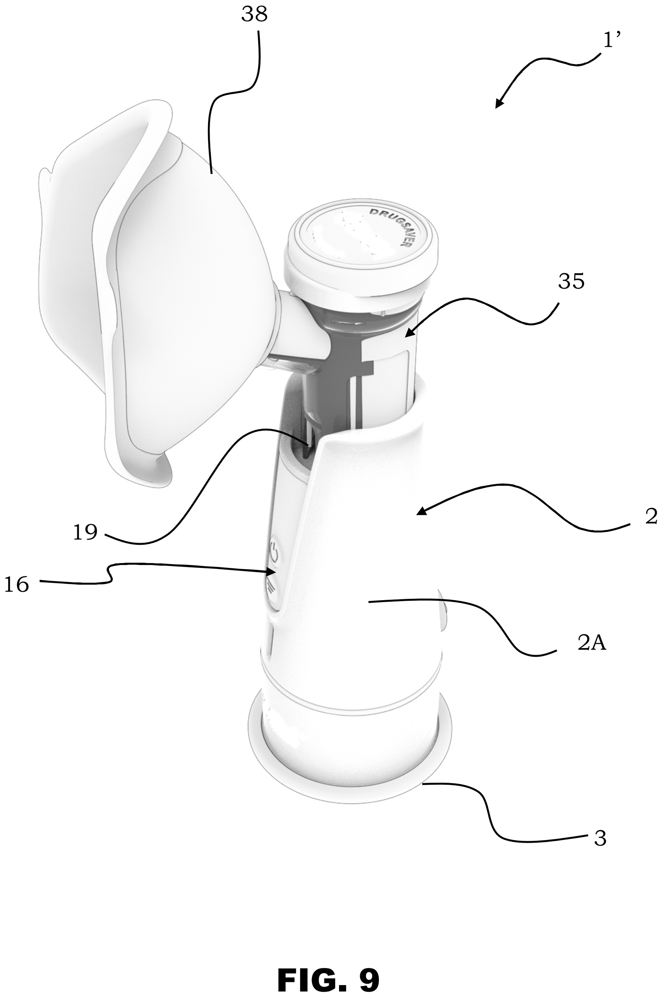

Figure 9 instead shows an embodiment that is substantially similar to the one depicted in Figure 7, but in which the nebulisation ampoule 35 further comprises a mask 38 for aerosol therapy.

The remaining features, in particular of the compressor unit 13, as well as the coupling features of the respiratory care device with the compressor unit 13 or with the main body 2, are similar both for the device 1 comprising the nasal douche 5 and for the device 1' comprising the nebulisation ampoule 35.

Advantageously, therefore, the compressor unit 13 may be used interchangeably with either a nasal douche or a nebulisation ampoule, with optimisation in terms of production, use, and dimensions.

Advantageously, the present solution allows air to be conveyed from a designated inlet to a designated outlet, passing from a defined and hermetic path. In this way, as previously mentioned, problems of pollution generally caused by internal components, such as DC motor brushes or wear and tear on electrical components, are avoided.

Furthermore, advantageously, the above described embodiment also allows having a pre-assembled and tested compressor unit that can be inserted and assembled in the body of the device with extreme simplicity, thus optimising production times even when the shape of the body of the device itself varies.

This preliminary assembly, in the event of changes in shape and size of the outer body, which may give rise to aesthetic variations, allows maintaining the optimisation of the components and assembly of the main and functional part of the device.

Still advantageously, the above described embodiment is suitable for frequent use, and is made to also optimise the cleaning and sterilisation steps, both in terms of simple disassembly and in terms of materials suitable for cleaning and disinfection operations.

Advantageously, the present device provides feedback on its own operation and battery via a light function of a ring located between body and bottom.

Moreover, advantageously, the preferred embodiment above described is particularly effective and less prone to maintenance interventions, working optimally for internal components.

Finally, advantageously, the present invention is particularly suitable for mass production, both in terms of expected costs and in terms of configuration of the components thereof.

A person skilled in the art will appreciate that the present embodiment may be subjected to several changes and variants, according to contingent and specific needs, all of them falling within the scope of protection of the invention defined by the appended claims.

For instance, as above described, nothing prevents variants with different shapes or materials from being provided, for instance for specific requirements related to specific users, or even simply.

## Claims

1. A cleaning device (1, 1') for nasal cavities comprising:
- a hollow main body (2) comprising an air inlet (11) and defining a housing space (4);
- a respiratory care device (5, 35) removably coupled to said main body (2);
- an activation element (16);
**characterized by** further comprising
a compressor unit (13) adapted to be inserted into said housing space (4) and comprising a hollow motor support (14) adapted to contain a compressor (15) and defined between a bottom (3) and an upper end (16) close to said respiratory care device (5, 35),
wherein said main body (2), said compressor unit (13), and said respiratory care device, when assembled, define an inner suction chamber (17) comprising a flow inlet (18), a compressor flow inlet (20), and a flow outlet (19), and
wherein said motor support (14) comprises a channel (21), preferably longitudinal, along an outer wall (22) of said motor support (14) defined between said air inlet (11) and said flow inlet (18) of said suction chamber (17).

2. The device (1, 1') according to claim 1, further comprising an air filter element (23) at said air inlet (11).

3. The device (1, 1') according to claim 2, wherein further an air filter cap (24) adapted to be inserted at said air filter element (23).

4. The device (1, 1') according to any one of claims 1 to 3, further comprising a control circuit (27) associated with said compressor unit (13).

5. The device (1, 1') according to claim 4, said motor support (14) being made of natural polypropylene PP, adapted to guide light signals of said control circuit (27) to an outer ring (34) of said motor support (14).

6. The device (1, 1') according to claim 4 or 5, further comprising a communication and/or power socket (30) and, preferably, a socket cap (31).

7. The device (1, 1') according to any one of claims 1 to 6, further comprising a lower motor support gasket (25) at a bottom of said motor support (14) and an upper motor support gasket (26) at said suction chamber (17).

8. The device (1, 1') according to any one of claims 1 to 7, wherein said activation element (16) comprises a first activation button (16A) on said main body (2) and a second activation button (16B) for selecting a nebulisation speed.

9. The device (1, 1') according to any one of claims 1 to 8, wherein said respiratory care device (5, 35) is couplable to said main body (2) by conical interference.

10. The device (1) according to any one of claims 1 to 9, wherein said respiratory care device is a nasal douche device (5) comprising:
- a reservoir (6);
- a recovery chamber (12);
- a nebulisation system (8);
- a nasal adapter (9) at an outlet (10) of said nebulisation system (8).

11. The device (1') according to any one of claims 1 to 9, wherein said respiratory care device is an aerosol therapy ampoule.

12. A compressor unit (13) for a cleaning device (1, 1') for nasal cavities, comprising a hollow motor support (14), a compressor (15) inserted in said motor support (14), and a channel (21), preferably longitudinal, along an outer wall (22) of said motor support (14).
